# FASCICULE DE BREVET EUROPEEN

(11) **EP 2 696 828 B1**
(45) Date de publication et mention de la délivrance du brevet: **31.05.2017**
(21) Numéro de dépôt: 12722417.8
(22) Date de dépôt: 13.04.2012
(51) Int. Cl.: A61F 13/02

(54) **PANSEMENT ADHESIF MINCE TRES ABSORBANT, SES UTILISATIONS POUR LE TRAITEMENT DES PLAIES CHRONIQUES**
HÖCHST SAUGFÄHIGER, DÜNNER KLEBEVERBAND UND SEINE VERWENDUNG ZUR BEHANDLUNG VON CHRONISCHEN WUNDEN
VERY ABSORBENT, THIN ADHESIVE DRESSING, AND USES THEREOF FOR THE TREATMENT OF CHRONIC WOUNDS

(30) Priorité: 15.04.2011 FR 1153327
(43) Date de publication de la demande: 19.02.2014
(73) Titulaire: Urgo Recherche Innovation et Développement, 21300 Chenove (FR)
(72) Inventeur: AUGUSTE, Stéphane, F-21490 Varois Et Chaignot (FR); PERNOT, Jean Marc, F-21000 Dijon (FR); DANEROL, Anne-Sophie, F-21000 Dijon (FR); CHARRE, Aurélie, F-69250 Montanay (FR)
(74) Mandataire: Chantraine, Sylvie Hélène
(86) Numéro de dépôt international: PCT/FR2012/050812
(87) Numéro de publication internationale: WO 2012/140378

(56) Documents cités:
- WO-A1-96/01659
- WO-A1-2004/064879
- WO-A1-2008/146529
- WO-A1-2010/147533
- WO-A2-2008/012443
- US-A1- 2009 216 168

## Description

La présente invention se rapporte à un pansement adhésif comprenant un support de protection imperméable respirant et un non tissé absorbant, ainsi qu'à son utilisation pour le soin des plaies chroniques ou aiguës, pour lesquelles la peau périlésionnelle est particulièrement fragile.

Les pansements absorbants adhésifs comprennent au moins une bordure adhésive, qui peut prendre la forme d'un « trottoir », et permet une fixation des pansements sur la peau qui entoure la plaie. Ces pansements, notamment lorsqu'ils sont utilisés pour le traitement des plaies chroniques particulièrement douloureuses, sont avantageusement conformables et minces afin de limiter les tensions que le pansement peut générer à la surface de la peau. Ils doivent aussi être très absorbants. Il est préférable d'utiliser, dans le soin de ce type de plaies, des pansements dont le trottoir est recouvert d'un adhésif siliconé, lequel est plus respectueux de la peau périlésionnelle fragile et sensible.

Il existe des pansements absorbants comprenant l'assemblage d'une mousse absorbante et d'un support de protection imperméable respirant. Le support de protection est imperméable aux fluides et aux micro-organismes pathogènes extérieurs, et perméable à la vapeur d'eau de manière à éviter le contact de la plaie avec des liquides et des bactéries et la macération de la plaie. L'épaisseur des mousses absorbantes est choisie selon la capacité d'absorption et la capacité de rétention des exsudats souhaitées, si bien qu'il faudra choisir une mousse très épaisse pour obtenir un pansement très absorbant.

Dans le produit Mepilex^{®} Border, il a donc été proposé d'assembler à la mousse un non tissé absorbant pour diminuer l'épaisseur totale du pansement sans diminuer sa capacité d'absorption. La fabrication de cette structure comprenant deux matériaux absorbants différents est cependant plus complexe à réaliser, et l'adhésif siliconé recouvre toute la surface du pansement.

Cependant, dans cette structure de pansement, le gel de silicone est en contact direct avec la plaie, et le fait que l'adhésif siliconé recouvre l'intégralité de la surface du pansement ne permet pas d'adapter de façon spécifique la couche d'interface qui peut recouvrir la surface de la mousse absorbante. Enfin dans un tel pansement, le support adhésivé n'intervient pas en tant qu'un trottoir.

Cette structure discontinue du gel de silicone permet d'éviter d'avoir à fixer le gel de silicone une fois réticulé à la couche absorbante du pansement. En effet, le gel de silicone une fois réticulé ne peut être durablement fixé à une mousse absorbante couramment utilisée dans la fabrication d'un pansement comprenant un trottoir adhésif.

Il existe donc un besoin de réaliser un pansement très absorbant dans lequel seule la surface du trottoir est recouverte d'un adhésif siliconé, et dont la structure comprend une compresse absorbante de manière à s'affranchir de l'emploi d'une mousse, pour obtenir un pansement encore plus mince.

Certaines compresses absorbantes présentent l'inconvénient de se désagréger et d'augmenter beaucoup de volume au fur et à mesure de l'absorption des exsudats, si bien qu'il est nécessaire de les envelopper. L'enveloppement de la compresse, qui est généralement un non tissé, doit répondre à certains critères dont l'accès des exsudats à la compresse et la fixation durable de l'enveloppe au support imperméable respirant utilisé. Ainsi, la taille de l'enveloppe doit permettre l'expansion du non tissé pendant l'absorption des exsudats de la plaie, et l'enveloppe ne doit pas se détacher du support quand elle devient humide au contact de la compresse chargée d'exsudats. Les risques de fuite de liquides, de délaminage des différentes couches constituant le pansement doivent être évités.

Dans la demande de brevet EP 358 412 par exemple, le pansement comprend un air-laid superabsorbant de pulpe de cellulose comprenant des fibres de polyéthylène et des particules superabsorbantes. Cette compresse est enveloppée dans un non tissé avant d'être collée par un adhésif acrylique au centre d'un support, qui peut être constitué de deux matériaux, un film plastique de polyéthylène collé par un adhésif acrylique à un non tissé de polyester ou de polypropylène. Ce pansement n'est cependant pas mieux adapté au soin des plaies chroniques ou aiguës, essentiellement pour deux raisons : le support en polyéthylène n'est pas respirant et l'adhésif acrylique appliqué sur la bordure peut endommager une peau périlésionnelle particulièrement fragile.

Dans le document US 6 096 942, la compresse absorbante est enveloppée dans un non tissé de fibres polyester. Les bordures du non tissé polyester sont soudées de manière à ce que la compresse reste confinée dans un compartiment fermé, sans être liée à l'enveloppe. La couche support du pansement est un film de polyuréthane flexible, perméable à la vapeur d'eau et imperméable aux liquides, enduit sur toute sa surface d'un adhésif acrylique pour coller à l'enveloppe en polyester. Ce pansement comprend un trottoir adhésif acrylique or, comme il a été expliqué plus haut, on préfère éviter le contact d'un adhésif acrylique avec la peau pour soigner les plaies chroniques. D'autres exemples de pansements sont décrits dans WO2010147533 et WO2008146529. La réalisation de pansements très absorbants adhésifs doit remplir un cahier des charges complexe et concilier des caractéristiques contradictoires. Les principaux critères que doivent vérifier un tel pansement sont essentiellement de présenter une bonne respirabilité tout en évitant les risques de fuites et de macération, d'être imperméable aux liquides et aux bactéries, d'être respirant (c'est-à-dire perméable à la vapeur d'eau), de garder sa cohésion quand il est retiré une fois chargé d'exsudats, d'être mince pour limiter la gêne que peut provoquer le pansement appliqué sur la peau, et d'être facile à fabriquer.

Le pansement doit également être facile à poser et rester en place le plus longtemps possible sans altérer la peau périlésionnelle, présenter une capacité d'absorption élevée, et ne pas altérer la cicatrisation de la plaie lors de son retrait. Le pansement doit aussi épouser la morphologie du patient et être compatible avec l'utilisation complémentaire d'un système de contention. Il doit aussi être le plus souple possible, et ne pas se rigidifier au cours de l'absorption des exsudats de la plaie.

Le choix des matériaux qui constituent le pansement, l'agencement des matériaux les uns par rapport aux autres, et les moyens d'assemblage de ces derniers sont très complexes pour obtenir toutes ces propriétés à la fois.

C'est l'objet de la présente invention que de fournir de nouveaux pansements absorbants adhésifs comprenant un non tissé absorbant et un support imperméable et haute perméabilité à la vapeur d'eau, plus faciles à fabriquer que les pansements de l'art antérieur de ce type, très minces, qui restent cohésifs en conditions sèches mais aussi humides, et dont le trottoir adhésif est enduit d'un gel de silicone adhésif.

L'invention propose en particulier un nouveau moyen de fixer un non tissé absorbant à un support comprenant une armature ajourée enduite de gel de silicone adhésif sans obturer les ouvertures de l'armature, sans diminuer les propriétés de respirabilité du support, sans diminuer les capacités d'absorption du non tissé absorbant, et sans diminuer la cohésion du pansement.

L'invention a ainsi pour objet un pansement absorbant adhésif comportant un non tissé absorbant, et un support de protection imperméable aux fluides et perméable à la vapeur d'eau, tel que :
- le support est constitué par l'assemblage d'un film continu et d'une armature ajourée enduite, sur au moins une de ses faces, de gel de silicone adhésif sans obturer les ouvertures de l'armature, ladite armature recouvrant l'intégralité de la surface du film,
- ledit pansement comprend en outre un voile non absorbant et un non tissé complémentaire lesquels sont fixés l'un à l'autre sur leur périphérie en enveloppant ledit non tissé absorbant, de préférence sans point de fixation avec ce dernier, et
- le voile non absorbant colle au gel de silicone adhésif enduit sur ladite armature.

Le voile non absorbant est un élément essentiel de l'invention car un support enduit d'un gel de silicone sur toute sa surface, et en particulier de façon discontinue, ne peut pas être fixé directement au non tissé absorbant enveloppé. Le non tissé absorbant enveloppé ne peut pas non plus être assemblé au support à l'aide d'un adhésif acrylate, lorsqu'il est enduit de gel de silicone.

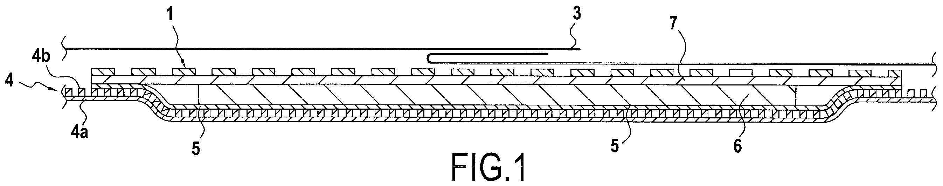

De tels pansements sont par exemple représentés en coupe droite dans la figure en annexe.

La figure représente un mode de mise en oeuvre de l'invention, dans lequel le non tissé absorbant 6 comprend des fibres cellulosiques et des particules de polymère superabsorbant.

Le non tissé absorbant 6 est successivement recouvert du côté faisant face à la plaie :
- d'un non tissé complémentaire 7, qui ne se délite pas lorsqu'il entre en contact avec les exsudats, et empêche les fibres et les particules de polymère superabsorbant contenu dans le non tissé absorbant 6 de polluer la plaie,
- d'une couche d'interface discontinue 1 constituée d'une composition à base d'élastomère contenant des particules d'hydrocolloïde, elle-même protégée par
- un protecteur pelable 3.

Le non tissé complémentaire est disposé du côté le plus proche de la plaie. La couche support 4 est constituée par l'assemblage d'un film imperméable aux liquides et perméable à la vapeur d'eau 4a, et d'une armature ajourée enduite d'un gel de silicone adhésif 4b sans obturer les ouvertures de l'armature. La couche support 4 est fixée au textile non tissé absorbant 6 par l'intercalage d'un voile non absorbant 5.

Dans ce mode de mise en oeuvre, le voile non absorbant 5 et le non tissé complémentaire 7 ont une dimension égale et sont soudés sur leurs bords, de manière à emprisonner le non tissé absorbant 6 dont la dimension est inférieure à celle du non tissé complémentaire et du voile non absorbant.

Le support est de préférence imperméable aux fluides et aux micro-organismes pathogènes extérieurs tout en assurant une perméabilité à la vapeur d'eau, de manière à éviter à la fois le contact de la plaie avec des liquides extérieurs et des bactéries et la macération de la plaie. On parle alors de « support imperméable et respirant ».

Le support est de préférence mince et souple, de manière à mieux épouser la forme du corps et suivre les mouvements sans risquer de se détacher. Le support est avantageusement conformable. Son épaisseur peut être comprise entre 100 et 600 µm, de préférence entre 250 et 500 µm.

Le support faisant partie du pansement selon l'invention est constitué d'un film continu 4a et d'une armature ajourée enduite d'un gel de silicone adhésif 4b sans obturer les ouvertures de l'armature. Le film est continu en ce sens qu'il n'a pas subi une étape de perforation.

Le film peut être remplacé par un complexe mousse/film ou un complexe textile/film. Parmi les films utilisables, on peut citer à titre d'exemple les films en polyétheruréthane, en polyétheramide, ou en polyétherester.

L'épaisseur du film est par exemple comprise entre 5 et 200 microns, de préférence entre 10 et 75 microns, de préférence encore entre 10 et 50 microns.

Le film continu est imperméable aux liquides et perméable à la vapeur d'eau. Le film continu peut être un film ou un complexe cité précédemment imperméable respirant tels ceux utilisés de façon courante pour la fabrication des pansements absorbants. Le film présente avantageusement un taux de transmission de vapeur d'eau (MVTR : Moisture Vapor Transmission Rate) supérieur à 3 000 g/m²/24 heures, de préférence supérieur ou égal à 7 000 g/m²/24 heures, de préférence encore supérieur ou égal à 10 000 g/m²/24 heures. L'armature ajourée enduite par un composé silicone sans obturer les ouvertures de l'armature. Cette armature ainsi enduite est avantageusement choisie de manière à ce que la valeur du taux de transmission de vapeur d'eau du support reste satisfaisante, notamment supérieure ou égale à 4 000 g/m²/24 heures, de préférence supérieure ou égal à 5 000 g/m²/24 heures. Une technique de mesure du taux de transmission de vapeur d'eau en contact liquide est décrite dans la norme NF-EN 13726-2 (Chapitre 3.3).

L'armature permet de rigidifier le support, de manière à ce qu'il ne s'enroule pas sur lui-même après retrait d'un protecteur pelable éventuellement utilisé pour protéger le non tissé complémentaire, la couche d'interface et/ou la bordure adhésive.

L'armature pourra être constituée de tout matériau ajouré tels un film perforé, un filet thermoplastique, un textile comme par exemple un tissé, un tricot, ou un non tissé, de préférence élastique pour une meilleure tenue du pansement sur la peau. Un film perforé sera par exemple en polyéthylène ou en polypropylène. Un textile tissé sera par exemple en polyéthylène téréphtalate ou en polyamide. Le grammage de l'armature est de préférence compris entre 10 et 500 g/m², par exemple entre 20 et 300 g/m². L'armature peut être enduite de gel de silicone sur une de ses faces, sur ses deux faces, voir sur toute sa surface. La taille des ouvertures de l'armature peut être comprise entre 0,1 et 5 mm, par exemple entre 0,5 et 3 mm. La surface ouverte de l'armature représente de préférence de 1 à 99%, de préférence de 25 à 90%, de préférence encore de 30 à 80%, de la surface du film continu, et la surface ouverte de l'armature une fois recouverte de gel de silicone représente de préférence de 10 à 99%, de préférence de 10 à 60%, de préférence encore de 25 à 75% de la surface du film continu.

Selon un mode de mise en oeuvre, on utilisera un tricot, de préférence un tricot enduit de gel de silicone sur toute sa surface sans obturer les ouvertures du tricot, qui pourra être avantageusement collé au film continu 4a.

Selon un autre mode de réalisation, l'armature est un film perforé enduit de gel de silicone sur une seule de ses faces sans obturer les perforations du film, par exemple un film de polyuréthane perforé, qui pourra être fixé au film continu par la chaleur, les ultrasons, la haute fréquence ou un adhésif.

Le gel de silicone adhésif est un composé de silicone dont la structure est réticulée. Le gel de silicone présente une cohésion telle qu'il ne laisse pas de résidus sur la peau et reste accroché à l'armature quand on retire le pansement. Il peut être fabriqué à partir de précurseurs de silicones qui réticulent après leur mise en contact suivant une réaction d'hydrosilylation ou de condensation. De tels systèmes sont connus de l'art antérieur, par exemple dans les documents EP-A-0 251 810, EP-A-0 300 620 ou US-A-4 921 704. Les mélanges de précurseurs décrits dans ces documents comprennent pour l'essentiel :
- un composant A qui comprend au moins une polydiméthylsiloxane substituée par un groupe vinyle à chacune de ses extrémités, et un catalyseur de platine, et
- un composant B de polydiméthylsiloxane qui comprend au moins deux groupes hydrogénosilanes.

La mise en présence des deux composants provoque une réaction de réticulation (crosslinking reaction) des deux polydiméthylsiloxanes fonctionnalisées qui se produit avantageusement à température ambiante et peut être accélérée par la chaleur.

Des additifs comme des pigments, des inhibiteurs ou des charges de remplissage peuvent être incorporés à l'un au moins des deux composants.

Les précurseurs du gel de silicone adhésif peuvent être choisis parmi les produits suivants : Silbione RT Gel^{®} 4712 A&B et Silbione RT Gel^{®} 4717 A&B de Bluestar Silicones, Wacker Silgel^{®} 612 de Wacker-Chemie GmbH, Nusil^{®} MED-6340, Nusil^{®} MED-6345, Nusil^{®} MED3-6300, ou Nusil^{®} MED12-6300 de Nusil Technology, et D-7-9800^{®} de Dow Corning.

Le gel de silicone est de préférence choisi de telle façon que le support présente un pouvoir adhésif sur peau, selon la méthode EN 1939, supérieur à 40 cN/cm, et préférence 45 cN/cm. Un échantillon de support de 20 mm de large et 150 mm de long est posé sur l'avant-bras. Au bout de 10 minutes, on mesure le pouvoir adhésif avec un dynamomètre à une vitesse de traction de 900 mm/min avec un angle de 90°.

Le gel de silicone est de préférence appliqué sur l'armature ajourée sans obturer les ouvertures de l'armature à raison d'un grammage compris entre 100 et 500 g/m², de préférence entre 150 et 250 g/m², de manière à assurer un compromis entre un taux de transmission de vapeur d'eau suffisant et une adhésion au film ou à la peau suffisante.

A titre d'exemple, on peut utiliser un support constitué de l'association d'un film en polyuréthane de 30 µm d'épaisseur qui présente un taux de transmission de vapeur d'eau de l'ordre de 7000 g/m²/24 heures, et d'un tricot de polyester 40 g/m² enduit d'un gel de silicone sur ses deux faces et sur toute sa surface à raison de 200 g/m². Ce support présente une épaisseur de l'ordre de 400 µm et une MVTR de l'ordre de 5000 g/m²/24 heures.

Le pansement selon l'invention comprend un voile intercalé entre le non tissé absorbant et le support, et destiné à les assembler. Ce moyen de fixation est nécessaire car la surface du support enduite d'un gel de silicone adhésif n'adhère pas de façon suffisante au non tissé absorbant, en particulier en milieu humide.

Le voile intercalé entre le non tissé absorbant et le support est un non tissé non absorbant de faible grammage. Le non tissé peut être tout type de non tissé couramment utilisé dans le domaine de l'hygiène et des pansements, notamment un non tissé filé lié (spun laid), cardé (carded) ou hydrolié (spun lace). Son grammage est de préférence compris entre 15 et 50 g/m², de préférence entre 20 et 40 g/m².

Le voile est non absorbant en ce sens qu'il ne contient pas de fibres absorbantes telles que la rayonne, la viscose, les dérivés de cellulose, et qu'il ne contient pas de particules absorbantes.

Il pourra être constitué de fibres de polyamide, de polyester, de polyuréthane et ou de polyoléfines. Selon un mode de réalisation le voile comprend des fibres de polyéthylène. Les fibres peuvent être monocomposants, ou bicomposants de type coeur/écorce ou côte-à-côte. On choisira par exemple un non tissé spun laid, de préférence de type spunbond.

Le voile non absorbant sera de préférence constitué de fibres hydrophobes, mais il pourra aussi être constitué de fibres hydrophiles et avoir subi un traitement pour le rendre hydrophobe. Le voile peut être constitué de plusieurs couches, dans la mesure où sa porosité est suffisante, la couche venant au contact du gel de silicone adhésif étant non absorbante et de préférence hydrophobe.

Le voile est fixé au non tissé absorbant sur toute sa surface, ou de préférence seulement sur sa périphérie par les technologies classiques de fixation tels la chaleur, les ultrasons, la haute fréquence, ou avec des adhésifs. Le voile est de préférence thermoscellable de manière à être fixé au non tissé complémentaire par la chaleur.

Dans le cadre de la présente invention, on utilisera par exemple un voile spun bond constitué de fibres de polyéthylène présentant un grammage compris entre 30 et 40 g/m², tel que celui commercialisé par la société Freudenberg sous la dénomination Vilmed^{®} LSO 1040 WEISS.

Un non tissé absorbant peut être choisi parmi les non tissés de fibres absorbantes comprenant des particules de polymère superabsorbant, et les non tissés comprenant des fibres superabsorbantes.

Le non tissé absorbant peut comprendre des fibres absorbantes telles que des fibres de cellulose, de rayonne ou de viscose. Le non tissé est préférablement obtenu par la méthode de fabrication par voie sèche connue sous le nom de voie aérodynamique ou "airlaid".

Tous les modes de liages couramment utilisés dans la technologie des non tissés pourront être employés pour fabriquer le non tissé absorbant: liage par enduction spray de latex, liage par incorporation de fibres ou de poudres thermoliantes puis traitement thermique, liage par association de ces deux techniques, liage par simple compression des fibres. Ce dernier mode de liage, ne faisant pas appel à l'incorporation de matériaux thermoliants ou de latex, sera préféré.

Selon un mode de mise en oeuvre de la présente invention, le non tissé absorbant incorpore des particules de polymères super-absorbants dans une proportion comprise entre 1 et 70% en poids, de préférence de 25 à 55% en poids, du poids total du non tissé. Le polymère superabsorbant peut être choisi parmi les polymères acryliques dont leurs sels tels que les polyacrylates de sodium.

Selon un mode préféré de la présente invention on utilisera un non tissé à base de particules de polymères superabsorbants et de fibres de cellulose sans l'incorporation de matériaux thermoliants ou de latex, qui sera recouvert sur chacune de ces faces par un voile cellulosique. Selon une autre variante de la présente invention on pourra aussi employer comme non tissé absorbant un non tissé constitué de deux voiles cellulosiques entre lesquels sont incorporés des particules de polymères super-absorbants seules ou en association avec des liants.

Le non tissé absorbant peut également comprendre des fibres superabsorbantes.

Dans le cadre de la présente invention, on préfère utiliser un non tissé présentant une épaisseur comprise entre 0,5 et 3 mm, et/ou un grammage compris entre 200 et 800 g/m² et/ou une absorption supérieure à 5000 g/m², de préférence encore supérieure ou égale à 15000 g/m². L'absorption pourra être mesurée selon la norme EDANA 440.1.99.

Des non tissés absorbants appropriés sont par exemple commercialisés par la société EAM Corporation sous la référence Novathin^{®}.

Selon une autre variante de la présente invention, le non tissé absorbant peut être remplacé par un autre matériau absorbant tel un tissé ou un tricot.

Le non tissé absorbant a de préférence des dimensions inférieures à celles du non tissé complémentaire et du voile non absorbant de manière à pouvoir augmenter de volume quand il absorbe les exsudats sans venir comprimer les parois de l'enveloppe, et risquer de l'ouvrir sous l'effet de la pression.

La surface du support est avantageusement supérieure à celle du non tissé absorbant enveloppé, si bien que le non tissé ne recouvre pas toute la surface du support adhésif. Selon la position du non tissé absorbant sur le support, la forme et la surface des bordures adhésives créées pourront être adaptées en fonction de l'anatomie de la partie du corps sur laquelle est destinée à être appliqué le pansement. Le non tissé absorbant enveloppé peut être centré sur le support, pour créer des bordures de largeur uniforme.

Le voile non absorbant et le non tissé complémentaire sont liés sur leurs bords latéraux en enveloppant le non tissé absorbant, de préférence sans point de fixation avec ce dernier.

Le non tissé complémentaire peut être tout type de non tissé couramment utilisé dans le domaine de l'hygiène et des pansements, notamment un non tissé filé lié (spun laid) ou cardé (carded). Sa structure pourra être renforcée par aiguilletage ou par ajout d'un polymère thermoliant sous forme de poudre ou de fibres. Son grammage est de préférence compris entre 15 et 200 g/m².

Le non tissé complémentaire peut être absorbant ou non absorbant. Au sens de « non absorbant » on entend qu'il ne contient pas de fibres absorbantes et qu'il ne contient pas de particules absorbantes.

Il pourra être constitué de fibres thermoplastiques choisies parmi les polyoléfines, les polyamides, les polyesters, les polyuréthanes et ou de polyoléfines. Il peut comprendre des fibres absorbantes telles que la rayonne, la viscose et les dérivés de cellulose. Il peut également comprendre des fibres superabsorbantes.

Les fibres peuvent être monocomposants, ou bicomposants de type coeur/écorce ou côte-à-côte.

Il peut être constitué de fibres hydrophiles ou hydrophobes, de préférence hydrophiles. Il peut aussi comprendre des fibres hydrophobes et avoir subi un traitement hydrophile.

Le non tissé complémentaire est avantageusement choisi parmi les non tissés hydrophiles, de préférence de fibres non absorbantes, de grammage compris entre 15 et 50 g/m², de préférence entre 20 et 40 g/m².

Selon un mode de mise en oeuvre, le non tissé complémentaire est de préférence un non tissé thermolié hydrophile non absorbant à base de fibres bicomposants polyethylène/polyester. Les références Sawabond® 4413 ou 4483 vendues par la société SANDLER en sont un exemple.

Selon un autre mode de mise en oeuvre, le non tissé complémentaire est choisi parmi les non tissés comprenant des fibres superabsorbantes et des fibres thermoliantes.

On pourra avantageusement déposer sur la surface du non tissé complémentaire, une couche d'interface 1 destinée à entrer en contact avec la plaie. La couche d'interface est discontinue pour permettre l'accès des exsudats au non tissé complémentaire; elle n'altère pas la plaie lors du retrait du pansement. La composition de la couche d'interface peut être hydrophobe, hydrophile ou amphiphile.

Selon un mode de réalisation de l'invention, la couche d'interface est micro-adhérente à la plaie, c'est-à-dire qu'elle permet de fixer provisoirement le pansement sur la plaie, et elle peut être retirée sans que la structure de la plaie ou de la peau périlésionnelle soit altérée.

On peut ainsi citer des compositions à base de polymères silicone en particulier des gels de silicone, des gels de polyuréthane, des compositions à base d'élastomère incluant des hydrocolloïdes, voire des hydrogels comme par exemple des hydrogels à base de poly(AMPS).

Dans le cadre de la présente invention, on préférera tout particulièrement utiliser une couche discontinue de composition contenant un élastomère, un plastifiant et des hydrocolloïdes. Cette couche d'interface favorise le processus de cicatrisation en maintenant un environnement humide au niveau de la plaie, et permet également de véhiculer des actifs, ce qui n'est pas le cas des interfaces siliconées.

L'élastomère peut être choisi parmi les polymères séquencés poly(styrène-oléfine-styrène) triblocs éventuellement associés à des copolymères diblocs. Les copolymères triblocs peuvent être des copolymères séquencés poly(styrène-éthylène-butylène-styrène) (en abrégé SEBS) vendus sous la dénomination KRATON^{®} G1651, KRATON^{®} G1654 ou KRATON^{®} G1652, ou des copolymères séquencés poly(styrène-éthylène-propylène-styrène) (en abrégé SEPS).

Parmi les composés plastifiants susceptibles d'être utilisés, on peut citer en particulier les huiles minérales, les polybutènes ou encore des dérivés de phtalate. D'une façon particulièrement préférée, on utilisera une huile plastifiante minérale choisie parmi les produits commercialisés sous les dénominations ONDINA^{®}933 et ONDINA^{®}919.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium connus sous la référence CMC Blanose^{®} 7H4XF, ainsi que les polymères synthétiques à base de sels de l'acide acrylique superabsorbants, comme par exemple les produits commercialisés par la société BASF sous la dénomination LUQUASORB^{®} 1003 ou par la société CIBA Specialty Chemicals sous la dénomination SALCARE^{®} SC91 ainsi que les mélanges de ces composés.

La composition à base d'élastomère incluant des hydrocolloïdes peut inclure si nécessaire un ou plusieurs antioxydants, ainsi que le tensioactif MONTANOX^{®} 80 ou le polymère SEPINOV^{®} EMT 10 tous les deux commercialisés par la société SEPPIC pour optimiser la vitesse de gélification, la mouillabilité ou le relargage d'actifs éventuellement présents dans la composition.

Si l'on souhaite que la couche d'interface soit micro-adhérente ou adhérente, la composition à base d'élastomère incluant des hydrocolloïdes contient un produit tackifiant qui peut être choisi parmi les résines tackifiantes, les polyisobutylènes de bas poids moléculaire ou leurs mélanges. De façon générale, on préférera l'utilisation de résines hydrogénées telles que les résines ESCOREZ^{®} de la série 5000 et tout particulièrement la résine ESCOREZ^{®} 5380.

La composition peut contenir des principes actifs ayant un rôle favorable dans le traitement de la plaie. Parmi les substances susceptibles d'être utilisées dans le cadre de la présente invention on peut, à titre d'exemples, citer :
- les antibactériens comme par exemple les dérivés d'argent tels les sels d'argent ou d'autres métaux (par exemple le sulfate, le chlorure ou le nitrate d'argent et la sulfadiazine argentique), les complexes d'argent ou d'autres métaux (par exemple les zéolithes argent tel l'alphasan, ou les céramiques), le métrodinazole, la néomycine, le Polymyxine B, les pénicillines (Amoxycilline), l'acide clavulanique, les tétracyclines, la Minocycline, la chlorotétracycline, les aminoglycosides, l'Amikacine, la Gentamicine ou les probiotiques;
- les antiseptiques tels la chlorhexidine, le triclosan, le biguanide, l'hexamidine, le thymol, le Lugol, la Povidone iodée, le Chlorure de Benzalkonium et de Benzethonium;
- les anti-douleurs tels que le Paracétamol, la Codéine, le Dextropropoxyphène, le Tramadol, la Morphine et ses dérivés, les Corticoïdes et dérivés;
- les anti-inflammatoires tels que les Glucocorticoïdes, les anti-inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique;
- les actifs favorisant la cicatrisation tels que le Rétinol, la Vitamine A, la Vitamine E, la N-acétyl-hydroxyproline, les extraits de *Centella Asiatica,* la papaïne, les huiles essentielles de thym, de niaouli, de romarin et de sauge, l'acide hyaluronique, les oligosaccharides polysulfatés et leurs sels (en particulier les oligosaccharides sulfatés synthétiques ayant 1 à 4 unités oses tels que le sel de potassium du sucrose octasulfaté ou le sel d'argent du sucrose octasulfaté), le sucralfate, l'Allantoïne, l'urée, la metformine, les enzymes (par exemple protéolitiques telles la streptokinase, la tripsine ou la collagénase), des peptides ou des inhibiteurs de protéases;
- les anesthésiques tels que la benzocaïne, la lidocaïne, la dibucaïne, le chlorhydrate de pramoxine, la bupivacaïne, la mepivacaïne, la prilocaïne, ou l'étidocaïne.

On préférera employer des compositions micro-adhérentes à base d'élastomère incluant des hydrocolloïdes qui, pour un total de 100 % en poids, comprennent:
0,05 à 1 % en poids d'antioxydant ;
10 à 60 % en poids de résine tackifiante ;
2 à 20 %, de préférence de 12 à 16 %, en poids de carboxyméthylcellulose de sodium ;
10 à 65 % en poids d'une huile minérale plastifiante ;
5 à 25 % en poids d'un polymère tribloc poly(styrène-éthylène-butylène-styrène) ou poly(styrène-éthylène-propylène-styrène) ; et
1 à 15 % en poids d'un copolymère constitué d'un sel de l'acide 2-méthyl-2-[(1-oxo-2-propényl)amino]-1-propane-sulfonique et de l'ester 2-hydroxyéthyle de l'acide propénoïque.

Une autre composition élastomère hydrocolloïde pourra comprendre, pour un total de 100 % en poids:
0,05 à 1 % en poids d'antioxydant ;
2 à 20 %, de préférence de 12 à 16 %, en poids de carboxyméthylcellulose de sodium ;
20 à 65 % en poids d'une huile minérale plastifiante ; et
3 à 25 % en poids d'un polymère tribloc poly(styrène-éthylène-butylène-styrène) ou poly(styrène-éthylène-propylène-styrène).

Le non tissé complémentaire éventuellement recouvert d'une couche d'interface pourra être protégé, au moins sur sa face destinée à venir en contact avec la plaie, d'une pellicule de protection qui pourra être retirée par pelage avant utilisation du pansement.

Le protecteur pelable 3 peut être constitué d'une ou plusieurs parties pelables avant usage. Ce protecteur recouvre toute la surface du pansement.

Ce protecteur pourra être tout matériau couramment utilisé comme protecteur par l'homme du métier dans le domaine des pansements. Il pourra se présenter par exemple sous forme de film par exemple un film de polyoléfine tels que le polyéthylène ou le polypropylène, un film de polyester mais également un papier. Ce film est avantageusement traité sur l'une au moins de ses faces par un composé siliconé comme un silane, un composé fluoré, ou un composé siliconé et fluoré.

Ce protecteur devra être adapté à la nature du gel de silicone adhésif. Ce protecteur devra également, le cas échéant, être adapté à la nature micro-adhérente de la couche d'interface.

Dans le cadre de la présente invention, on préférera en particulier l'utilisation d'un protecteur en deux parties, comme indiqué sur la figure.

Le protecteur pelable est de préférence doté d'une épaisseur comprise entre 10 et 100 µm, par exemple de l'ordre de 50 µm.

Le produit commercialisé sous la référence Silflu® M1R88001 par la société Siliconature peut avantageusement être utilisé comme tel.

L'épaisseur totale du pansement est avantageusement comprise entre 3 et 5 mm, de préférence de l'ordre de 4 à 4,5 mm.

Le pansement de l'invention est avantageusement indiqué pour le traitement de toutes les plaies exsudatives chroniques (escarre, ulcère, comme par exemple l'ulcère du pied du diabétique) et aiguës (brûlure du 2ème degré, dermabrasion, plaie traumatique, plaie post-opératoire).

Le pansement de l'invention particulièrement indiqué pour le traitement des plaies lorsque la peau péri-lésionnelle est fragilisée.

Dans le cadre de la présente invention, on préférera l'utilisation d'un pansement à coins arrondis pour éviter un décollement prématuré.

Le pansement de l'invention peut se présenter sous forme de pansements individuels de petite dimension ou de dimension plus importante. Las pansements seront conditionnés individuellement dans une enveloppe scellée assurant une conservation en milieu stérile.

La présente invention a également pour objet un procédé de fabrication du pansement décrit précédemment qui consiste à réaliser le support, à fixer le voile non absorbant et le non tissé complémentaire l'un à l'autre en enveloppant le non tissé absorbant, de préférence sans point de fixation avec ce dernier, puis à assembler au support le non tissé absorbant enveloppé du côté du voile non absorbant.

Selon un mode de réalisation, le non tissé absorbant enveloppé est assemblé au support en mettant en contact le voile non absorbant et le côté du support recouvert de gel de silicone adhésif.

Dans une première étape, on réalise le support par enduction de l'armature et assemblage de l'armature enduite au film continu.

Le gel de silicone sera enduit sur l'armature en utilisant une des techniques d'enduction couramment employée par l'homme de l'art.

Selon la version préférée de la présente invention, le gel de silicone est enduit sur les deux faces de l'armature, qui est assemblée au film continu alors que la réticulation du gel n'est pas complète, de manière à assurer la cohésion entre le film et l'armature ajourée. Dans ce mode de mise en oeuvre, aucun adhésif n'est nécessaire pour faire adhérer l'armature au film imperméable aux liquides et perméable à la vapeur d'eau.

Dans cette version, le support peut être fabriqué selon la succession d'étapes suivantes :
- l'armature est recouverte sur ses deux faces d'un mélange des précurseurs du gel de silicone,
- l'armature est assemblée au film par exemple par calandrage, et
- la réticulation du gel de silicone est provoquée ou accélérée une fois que l'armature et le film ont été assemblés, en plaçant par exemple le support dans un four.

L'armature est par exemple plongée dans le mélange des précurseurs du gel de silicone, puis essorée dans un poste de laminage entre deux rouleaux. Une soufflerie permet de reconstituer les ouvertures de l'armature pour enlever le surplus de gel de silicone.

Dans une autre version, le gel de silicone est enduit sur une des deux faces de l'armature, et l'autre face est fixée au film continu par un adhésif. Elle peut aussi être fixée par la chaleur, les ultra-sons ou la haute fréquence et dans ce cas, l'armature et/ou le film continu pourront être thermoplastiques, de manière à les thermosceller.

Le support sera protégé en recouvrant sa face adhésive par une couche ou pellicule de protection. Cette couche de protection pourra être par exemple du papier ou un film de polyester.

Dans une deuxième étape, le non tissé absorbant est enveloppé.

Une bobine de voile et une bobine de non tissé absorbant sont déroulées puis détendues, avant d'être superposées. On procède ensuite à une découpe du non tissé absorbant dans son épaisseur, de préférence sous formes de carrés, puis ils sont déposés sur le voile. Une bobine de non tissé complémentaire est déroulée et détendue, puis la couche de non tissé complémentaire est superposé à l'ensemble constitué de la couche de voile et de la couche de non tissé absorbant préalablement découpé. Le voile non absorbant et le non tissé complémentaire sont fixés ensemble seulement sur leur périphérie par la chaleur, les ultrasons, la haute fréquence ou avec un adhésif. On procède par exemple au thermoscellage du voile et du non tissé complémentaire sur un tracé éloigné des bords du non tissé absorbant de manière à créer une enveloppe de taille suffisante qui permet au non tissé absorbant de gonfler lors de l'absorption des liquides.

Le voile et le non tissé complémentaire sont de préférence scellés par la chaleur ou les ultra-sons. La température de scellage sera par exemple comprise entre 80 et 150°C, de préférence encore entre 90 et 120 °C, notamment de l'ordre de 110°C. On procède à la fixation du voile et du non tissé complémentaire par exemple sur une largeur de 1 à 3 mm.

Le non tissé absorbant enveloppé est ensuite échenillé puis assemblé au support fabriqué précédemment, du côté de sa surface adhésive, dans un poste de laminage en appliquant une pression de consigne allant de 0 à 10 bars, de préférence de 0 à 6 bars. Le poste de laminage est avantageusement un poste de tirage.

Avant de réaliser la découpe, on recouvre l'ensemble support/non tissé enveloppé, du côté du non tissé complémentaire destiné à venir en contact avec la plaie, avec une pellicule de protection qui pourra être retirée par pelage avant utilisation du pansement.

Si le non tissé complémentaire a été préalablement recouvert d'une couche d'interface et d'un protecteur pelable provisoire, ledit protecteur provisoire sera retiré pour recouvrir l'ensemble support/ non tissé enveloppé /couche d'interface, du côté de la couche d'interface destinée à venir en contact avec la plaie, avec une pellicule de protection qui pourra être retirée par pelage avant utilisation du pansement.

Lorsque l'on souhaite fixer le voile non absorbant au non tissé complémentaire avec un adhésif, le voile complémentaire déroulé et détendu est enduit d'adhésif avant d'être calandré avec l'ensemble constitué de la couche de voile et de la couche de non tissé absorbant préalablement découpé.

La surface du non tissé complémentaire destinée à venir en contact avec la plaie, éventuellement recouverte d'une couche d'interface, pourra être protégée en la recouvrant d'une pellicule de protection qui pourra être retirée par pelage avant utilisation du pansement.

Une fois le voile non absorbant et le non tissé complémentaire fixés l'un à l'autre, en enveloppant le non tissé absorbant, de préférence sans point de fixation avec ce dernier, l'ensemble est de préférence assemblé au support par le même procédé de laminage que décrit précédemment. Le choix d'un voile non absorbant conformément à la présente invention permet avantageusement d'assembler le support et le voile sans qu'il soit nécessaire d'avoir recours à un adhésif acrylique comme dans l'art antérieur. Le support et le voile non absorbant restent solidaires lorsque le pansement usagé est retiré, ou décollé de la peau.

Selon un mode de réalisation, le voile non absorbant subit avantageusement un traitement Corona, de préférence juste après son déroulage. Le traitement Corona permet d'augmenter l'accroche du voile sur le support enduit de gel de silicone adhésif. Dans ce mode de réalisation, le voile peut être constitué de fibres de polyéthylène.

Le non tissé absorbant enveloppé peut être recouvert d'une couche d'interface sur la surface du non tissé complémentaire destinée à venir en contact avec la plaie, avant d'être complexé au différent élément constituant la compresse.

Une couche d'interface à base d'élastomère contenant des hydrocolloïdes décrite plus haut peut être fabriquée suivant un procédé hot melt bien connu de l'homme du métier, par malaxage à chaud des différents constituants à une température comprise entre 90 et 160°C et de préférence entre 110 et 140°C. On préfère tremper un cylindre gravé dans la composition préalablement malaxée à chaud, puis la composition encore chaude est démoulée et transférée sur le non tissé complémentaire. L'application de la composition encore chaude sur le non tissé complémentaire permet d'optimiser l'accroche de la couche d'interface. Un protecteur provisoire peut être placé sur le non tissé complémentaire recouvert de la couche d'interface, pour procéder à l'étape de calandrage avec le support adhésif.

Le protecteur provisoire est retiré après la fixation du non tissé complémentaire recouvert de la couche d'interface au support, pour appliquer un protecteur pelable qui sera retiré avant l'application du pansement sur la plaie.

L'invention est illustrée par l'exemple suivant.

### Exemple 1: Préparation d'un pansement absorbant

On fabrique un pansement avec trottoir comprenant un non tissé absorbant enveloppé dans un voile non absorbant de polyéthylène et un non tissé complémentaire hydrophile. Une couche d'interface microadhérente est déposée sur la surface du non tissé complémentaire destinée à entrer en contact avec la plaie.

Les matériaux suivants sont utilisés :
- Le support est un tricot de polyester 40 g/m² enduit sur ses deux faces et sur toute sa surface d'un gel de silicone adhésif (200 g/m²), qui a été laminé sur un film de polyuréthane de 30 µm d'épaisseur. Cette couche support présente une épaisseur de l'ordre de 300 µm et une MVTR de l'ordre de 5000 g/m²/24 heures.
- Le voile de polyéthylène est un non tissé 40 g/m² vendu sous la référence Vilmed^{®} LSO 1040 WEISS par Freudenberg.
- Le non tissé absorbant est un airlaid (400 g/m²) contenant 50 % de polymère superabsorbant de chez EAM Corporation vendu sous la référence Novathin^{®}.
- Le non tissé complémentaire est un non tissé hydrophile de référence Sawabond^{®} 4483 vendu par la société SANDLER.
- Le protecteur pelable comprend des ailettes en PET fluoré de 50 nm, fourni par SILICONATURE sous la référence SILFLU^{®} M1R88001.

### Préparation de la couche d'interface et enduction sur le non tissé complémentaire :

On prépare la composition suivante, exprimée en pourcentage en poids par rapport au poids total:
- Huile minérale commercialisée par la société Shell sous la dénomination Ondina^{®}919 : 39,7%
- Sel de sodium de carboxyméthylcellulose commercialisé par la société AQUALON sous la dénomination CMC Blanose^{®} 7H4XF : 14,8%
- Copolymère séquencé de poly(styrène-éthylène-butylène) commercialisé par la société KRATON sous la dénomination KRATON^{®} G 1651E : 4,7%
- Antioxydant commercialisé sous la dénomination IRGANOX^{®} 1010 par la société CIBA SPECIALTY CHEMICALS : 0,2%.
- Copolymère de sel de l'acide 2-méthyl-2[(1-oxo-2-propényl)amino]-1-propanesulfonique et de l'ester 2-hydroxyéthyle de l'acide propénoïque (agent de relargage) commercialisé par la société SEPPIC sous la dénomination SEPINOV^{®} EMT 10 : 5%.
- Résine tackifiante commercialisée par la société EXXON CHEMICALS sous la dénomination ESCOREZ^{®} 5380 : 35,6%.

On a introduit l'huile minérale, l'hydrocolloïde, et l'élastomère puis l'antioxydant et l'agent de relargage et enfin la résine tackifiante portés à une température comprise entre 100 et 110°C dans un malaxeur MEL G-40, de manière à obtenir un mélange homogène.

Le mélange précédent est enduit de façon discontinue en une quantité de 170 g/m² (± 40) sur le non tissé complémentaire.

### Assemblage des couches :

On découpe des carrés de non tissé complémentaire et de voile de polyéthylène que l'on soude sur une largeur de 2 mm avec une soudeuse manuelle AMIS sur un seul côté. Le voile de polyéthylène a préalablement subi un traitement Corona dans les conditions suivantes :
- Puissance du générateur : 570 watts
- Nombre d'électrodes/largeur : 3/0,25 m
- Réglage de l'entrefer : 2 mm
- Vitesse de défilement : 2 m/minute

Le non tissé absorbant est ensuite inséré entre le non tissé complémentaire et le voile.

Les trois autres côtés du non tissé complémentaire et du voile de polyéthylène sont soudés dans les mêmes conditions que précédemment de manière à former un carré de 8 x 8 cm, puis les bords du complexe assemblé sont découpés.

Le support est découpé selon un carré de 15 x 15 cm, puis assemblé au complexe précédant par calandrage avec un rouleau de 10 kg dans deux directions perpendiculaires.

On procède ensuite à la découpe du pansement final.

## Revendications

1. Pansement absorbant adhésif comportant un non tissé absorbant (6) et un support de protection imperméable aux fluides et perméable à la vapeur d'eau (4), **caractérisé en ce que** :
- le support est constitué par l'assemblage d'un film continu (4a) et d'une armature ajourée enduite, sur au moins une de ses faces, de gel de silicone adhésif (4b), sans obturer les ouvertures de l'armature, ladite armature recouvrant l'intégralité de la surface du film,
- **en ce que** ledit pansement comprend en outre un voile non absorbant (5) et un non tissé complémentaire (7) lesquels sont fixés l'un à l'autre sur leur périphérie en enveloppant ledit non tissé absorbant, de préférence sans point de fixation avec ce dernier, et
- **en ce que** ledit voile non absorbant (5) colle au gel de silicone adhésif (4b) enduit sur ladite armature.

2. Pansement selon la revendication précédente, **caractérisé en ce que** le voile non absorbant (5) présente un grammage compris entre 15 et 50 g/m².

3. Pansement selon l'une des revendications précédentes, **caractérisé en ce que** le grammage du gel de silicone adhésif est compris entre 100 et 500 g/m².

4. Pansement selon l'une des revendications précédentes, **caractérisé en ce que** le non tissé complémentaire (7) est choisi parmi les non tissés hydrophiles à base de fibres non absorbantes dont le grammage est compris entre 15 et 50 g/m².

5. Pansement selon l'une des revendications 1 à 3, **caractérisé en ce que** le non tissé complémentaire (7) est choisi parmi les non tissés comprenant des fibres superabsorbantes et des fibres thermoliantes.

6. Pansement selon l'une des revendications précédentes, **caractérisé en ce que** l'armature ajourée (4b) est un tricot enduit de gel de silicone adhésif sur ses deux faces et sur toute sa surface sans obturer les ouvertures du tricot.

7. Pansement l'une des revendications 1 à 5, **caractérisé en ce que** l'armature ajourée (4b) est un film perforé enduit de gel de silicone adhésif sur une seule de ses faces sans obturer les perforations du film.

8. Pansement l'une des revendications 1 à 5, **caractérisé en ce que** le non tissé absorbant (6) est centré sur le support (4) pour créer des bordures adhésives.

9. Procédé de fabrication d'un pansement selon l'une des revendications précédentes consistant à réaliser le support (4), à fixer le voile non absorbant (5) et le non tissé complémentaire (7) l'un à l'autre en enveloppant le non tissé absorbant (6), de préférence sans point de fixation avec ce dernier, puis à assembler au support (4) le non tissé absorbant (6) enveloppé du côté du voile non absorbant (5).

10. Procédé selon la revendication 9, **caractérisé en ce que** le voile non absorbant (5) et le non tissé complémentaire (7) sont fixés ensemble seulement sur leur périphérie par la chaleur, les ultrasons, la haute fréquence ou avec un adhésif.

11. Procédé selon la revendication 9 ou 10, **caractérisé en ce que** le support (4) est assemblé au non tissé absorbant (6) enveloppé dans un poste de laminage en appliquant une pression allant de 0 à 10 bars.

12. Procédé selon l'une des revendications 9 à 11, **caractérisé en ce que** le voile non absorbant (5) subit un traitement Corona, juste après son déroulage.

## Patentansprüche

1. Saugfähiger Klebeverband, umfassend ein absorbierendes Vlies (6) und einen flüssigkeitsundurchlässigen und wasserdampfdurchlässigen Schutzträger (4), **dadurch gekennzeichnet, dass**:
- der Träger durch Zusammenfügen eines durchgehenden Films (4a) und einer durchbrochenen Einlage, die auf wenigstens einer ihrer Seiten mit Haftsilikongel (4b) überzogen ist, ohne die Öffnungen der Einlage zu verschließen, gebildet ist, wobei die Einlage die gesamte Oberfläche des Films bedeckt,
- dass der Verband ferner einen nicht absorbierenden Flor (5) und ein ergänzendes Vlies (7) umfasst, die an ihrem Umfang unter Umschließen des absorbierenden Vlieses aneinander befestigt sind, vorzugsweise ohne Befestigungsstelle mit letzterem, und
- dass der nicht absorbierende Flor (5) an dem auf die Einlage aufgebrachten Haftsilikongel (4b) klebt.

2. Verband nach dem vorhergehenden Anspruch, **dadurch gekennzeichnet, dass** der nicht absorbierende Flor (5) ein Flächengewicht zwischen 15 und 50 g/m² aufweist.

3. Verband nach einem der vorhergehenden Ansprüche, **dadurch gekennzeichnet, dass** das Flächengewicht des Haftsilikongels zwischen 100 und 500 g/m² beträgt.

4. Verband nach einem der vorhergehenden Ansprüche, **dadurch gekennzeichnet, dass** das ergänzende Vlies (7) aus den hydrophilen Vliesen auf Basis von nicht absorbierenden Fasern, deren Flächengewicht zwischen 15 und 50 g/m² beträgt, ausgewählt ist.

5. Verband nach einem der Ansprüche 1 bis 3, **dadurch gekennzeichnet, dass** das ergänzende Vlies (7) aus den Vliesen, die höchst saugfähige Fasern und warmverbindende Fasern umfassen, ausgewählt ist.

6. Verband nach einem der vorhergehenden Ansprüche, **dadurch gekennzeichnet, dass** die durchbrochene Einlage (4b) ein Gewirk ist, das auf seinen beiden Seiten und auf seiner gesamten Oberfläche mit Haftsilikongel überzogen ist, ohne die Öffnungen des Gewirks zu verschließen.

7. Verband nach einem der Ansprüche 1 bis 5, **dadurch gekennzeichnet, dass** die durchbrochene Einlage (4b) ein perforierter Film ist, der auf nur einer seiner Seiten mit Haftsilikongel überzogen ist, ohne die Löcher des Films zu verschließen.

8. Verband nach einem der Ansprüche 1 bis 5, **dadurch gekennzeichnet, dass** das absorbierende Vlies (6) auf dem Träger (4) zentriert ist, um Haftumrandungen zu schaffen.

9. Verfahren zur Herstellung eines Verbands nach einem der vorhergehenden Ansprüche, das darin besteht, den Träger (4) herzustellen, den nicht absorbierenden Flor (5) und das ergänzende Vlies (7) unter Umschließen des absorbierenden Vlieses (6) aneinander zu befestigen, vorzugsweise ohne Befestigungsstelle mit letzterem, anschließend das umschlossene absorbierende Vlies (6) auf der Seite des nicht absorbierenden Flors (5) mit dem Träger (4) zu verbinden.

10. Verfahren nach Anspruch 9, **dadurch gekennzeichnet, dass** der nicht absorbierende Flor (5) und das ergänzende Vlies (7) nur an ihrem Umfang durch Hitze, Ultraschall, Hochfrequenz oder mit einem Klebemittel aneinander fixiert werden.

11. Verfahren nach Anspruch 9 oder 10, **dadurch gekennzeichnet, dass** der Träger (4) mit dem umschlossenen absorbierenden Vlies (6) in einer Walzstation unter Anlegen eines Druckes von 0 bis 10 Bar zusammengefügt wird.

12. Verfahren nach einem der Ansprüche 9 bis 11, **dadurch gekennzeichnet, dass** der nicht absorbierende Flor (5) direkt nach seinem Abwickeln einer CoronaBehandlung unterzogen wird.

## Claims

1. An adhesive absorbent dressing comprising an absorbent nonwoven (6) and a protective substrate (4) that is impermeable to fluids, but permeable to water vapor, **characterized in that**:
- the substrate is formed by assembling a continuous film (4a) and an openwork reinforcement that is coated, on at least one of its faces, with adhesive silicone gel (4b) without blocking the openings in the reinforcement, said reinforcement covering the entire surface of the film,
- **in that** said dressing also comprises a non-absorbent web (5) and a complementary nonwoven (7) which are secured to one another along their periphery while encasing said absorbent nonwoven without being secured at any point to the latter, and
- **in that** said non-absorbent web (5) adheres to the adhesive silicone gel (4b) that is coated onto said reinforcement.

2. The dressing as claimed in the preceding claim, **characterized in that** the non-absorbent web (5) has a grammage of between 15 and 50 g/m².

3. The dressing as claimed in either of the preceding claims, **characterized in that** the grammage of the adhesive silicone gel is between 100 and 500 g/m².

4. The dressing as claimed in one of the preceding claims, **characterized in that** the complementary nonwoven (7) is chosen from hydrophilic nonwovens based on non-absorbent fibers, the grammage of which is between 15 and 50 g/m².

5. The dressing as claimed in one of claims 1 to 3, **characterized in that** the complementary nonwoven (7) is chosen from nonwovens comprising superabsorbent fibers and thermal bonding fibers.

6. The dressing as claimed in one of the preceding claims, **characterized in that** the openwork reinforcement (4b) is a knit that is coated with adhesive silicone gel on both its faces and on its entire surface without blocking the openings of the knit.

7. The dressing as claimed in one of claims 1 to 5, **characterized in that** the openwork reinforcement (4b) is a perforated film that is coated with adhesive silicone gel on just one of its faces, without blocking the perforations of the film.

8. The dressing as claimed in one of claims 1 to 5, **characterized in that** the absorbent nonwoven (6) is centered on the substrate (4), so as to create adhesive borders.

9. A process for manufacturing a dressing as claimed in one of the preceding claims, which consists in producing the substrate (4), in securing the non-absorbent web (5) and the complementary nonwoven (7) to one another while encasing the absorbent nonwoven (6), preferably without them being secured at any point to the latter, and then in assembling the encased absorbent nonwoven (6) to the substrate (4), on the side of the non-absorbent web (5).

10. The process as claimed in claim 9, **characterized in that** the non-absorbent web (5) and the complementary nonwoven (7) are secured together only along their periphery via heat, ultrasound, high frequency or with an adhesive.

11. The process as claimed in claim 9 or 10, **characterized in that** the substrate (4) is assembled to the encased absorbent nonwoven (6) in a lamination station by applying a pressure ranging from 0 to 10 bar.

12. The process as claimed in one of claims 9 to 11, **characterized in that** the non-absorbent web (5) undergoes a Corona treatment, just after it has been unwound.
